# EUROPÄISCHE PATENTSCHRIFT

(11) **EP 1 676 819 B1**
(45) Veröffentlichungstag und Bekanntmachung des Hinweises auf die Patenterteilung: **18.02.2009**
(21) Anmeldenummer: 05026448.0
(22) Anmeldetag: 05.12.2005
(51) Int. Cl.: C02F 11/04, C12P 5/02

(54) **Verfahren zur umweltverträglichen Behandlung von Klärschlamm sowie Anordnung umfassend eine Kläranlage**
An environmentally compatible process for the treatment of organic sludge and a waste water treatment plant
Procédé pour le traitement des boues organiques sans danger pour l'environnement et station d'épuration des eaux

(30) Priorität: 31.12.2004 DE 102004063672
(43) Veröffentlichungstag der Anmeldung: 05.07.2006
(73) Patentinhaber: EISENMANN Anlagenbau GmbH & Co. KG, 71032 Böblingen (DE)
(72) Erfinder: Nacke, Oliver, 31840 Hess. Oldendorf (DE); Link, Kersten, 71120 Grafenau (DE)
(74) Vertreter: Ostertag, Ulrich

(56) Entgegenhaltungen:
- EP-A- 1 522 529
- DE-A1- 3 117 461
- DE-A1- 3 138 452
- DE-A1- 10 107 712
- DE-A1- 19 511 734
- DE-A1- 19 615 551
- DE-A1- 19 828 889
- US-A1- 2003 173 291
- BERGMANN D: "GEMEINSAME BEHANDLUNG VON BIOABFALL UND KLAERSCHLAMM" KORRESPONDENZ ABWASSER, ABWASSERTECHNISCHE VEREINIGUNG, ST. AUGUSTIN, DE, Bd. 44, Nr. 10, Oktober 1997 (1997-10), Seiten 1778-1780,1783, XP000731364 ISSN: 0341-1540

## Beschreibung

Die vorliegende Erfindung betrifft ein Verfahren zur umweltverträglichen Behandlung von Klärschlamm nach dem Oberbegriff des Patentanspruchs 1 sowie eine Anordnung umfassend eine Kläranlage mit einem Faulturm zur anaeroben Stabilisierung anfallenden Klärschlamms nach dem Oberbegriff des Patentanspruchs 5.

Bekannte Kläranlagen verfügen über eine mechanische Stufe mit einem Absetzbecken, in dem sich Klärschlamm absetzt. Je nach Art der Kläranlage besteht der Klärschlamm aus den absetzbaren Stoffen im Abwasser (Primärschlamm) und dem Überschußschlamm aus den Belebungsbecken bzw. Tropfkörpern. Dieses Gemisch enthält organische Substanz (ca. 60-80% des Trockenschlamms) aus den Stoffgruppen Kohlenhydrate, Fette und Eiweiße. Der Klärschlamm ist daher ein idealer Nährboden für Mikroorganismen.

Man unterscheidet zwischen aeroben und anaeroben Mikroorganismen (Bakterien). Von den aeroben Mikroorganismen werden die organischen Inhaltsstoffe in einem Stoffwechselprozeß zu CO₂ und H₂O "veratmet" (Oxidation). Anaerobe Mikroorganismen können hingegen die hochmolekularen Substanzen nicht veratmen sondern nur "vergären" (Reduktion). Dabei entstehen hauptsächlich Methan (CH₄) und Kohlendioxid (CO₂).

Da im Rohschlamm stets Mikroorganismen in großer Zahl enthalten sind, setzen im abgelagerten Rohschlamm spontan anaerobe, mikrobiologische Umsetzungsprozesse ein, die aufgrund der dabei neben Methan und Kohlendioxid zusätzlich entstehenden Schwefelwasserstoffverbindungen zu Geruchsbelästigungen führen und außerdem die Entwässerbarkeit des Schlamms verschlechtern. Zur Beseitigung dieser kritischen Eigenschaften der Rohschlämme wird eine sogenannte Schlammstabilisierung und anschließend eine Abtrennung des Schlammwassers durchgeführt.

Unter anaerober Stabilisierung versteht man den biologischen Abbau organischer Stoffe durch anaerobe Mikroorganismen. Dabei gilt nach DIN 4045 ein Schlamm dann als "stabilisiert", wenn durch eine entsprechende Behandlung mindestens eines der beiden Hauptziele der Schlammstabilisierung, nämlich weitgehende Verringerung der geruchsbildenden Inhaltsstoffe und weitgehende Verringerung der organischen Schlammfeststoffe, erreicht wurde. Neben diesen Hauptzielen sind als Nebenziele noch die Verbesserung der Entwässerbarkeit, die Verminderung der Krankheitserreger und gegebenenfalls die Gewinnung von Energie erwünscht.

Organische Substanz ist stabil, wenn sie nicht mehr durch mikrobiellen Abbau zersetzt werden kann. Absolute Stabilität kann nur durch vollständige Mineralisierung, also durch Verbrennung oder Verschwelung erreicht werden. Biologische Abbauprozesse verlaufen hingegen asymptotisch, nähern sich also einem Gleichgewichtszustand an, ohne ihn jemals ganz erreichen zu können.

Für die Praxis ist eine relative Stabilisierung ausreichend, die sich dadurch auszeichnet, daß keine üblen Gerüche mehr freigesetzt werden. Die biologischen Stabilisierungsverfahren beruhen darauf, den Abbau möglichst kontrolliert, schnell und unter Vermeidung unangenehmer Emissionen so weitgehend ablaufen zu lassen, daß das Produkt anschließend nur noch sehr langsam weiter zersetzt werden kann. Klärschlamm der kommunalen Kläranlagen besteht typischerweise noch aus ca. 40-50% organischen Substanzen.

Im Vergleich zu aeroben Stabilisierungsverfahren bieten anaerobe Verfahren verschiedene Vorteile. Der Energiebedarf für eine Sauerstoffversorgung entfällt, es entsteht nur wenig verbleibende Bakterienmasse, der Abbau erfolgt in einem geschlossenen Behälter, dem Faulturm, so daß keine Geruchsbelästigung auftreten kann, und das anfallende Klärgas mit einem Heizwert von etwa 6000 kcal/m³ kann als wertvoller Energieträger genutzt werden.

Dennoch setzen kleine und mittlere Kläranlagen bis zu einer Größenordnung von ca. 30.000 Einwohnergleichwerten heute hauptsächlich eine aerobe Stabilisierung ein. Dies liegt zum einen daran, daß sich die Investitions- und Betriebskosten für die relativ kleinen Blockheizkraftwerke zur Verstromung des anfallenden Klärgases nicht rentieren. Da die Energie aus dem Klärgas bei solchen Anlagen zur Deckung des Versorgungsbedarfes nicht ausreicht, müßte mit Heizöl oder Erdgas zugeheizt werden, was die Energiekosten steigern und einen Betrieb unrentabel machen würde. Zum anderen verfügen Kläranlagen mit anaerober Stabilisierung besonders im Winter nicht über genügend eigene Wärme aus einer Verstromung des vorhandenen Klärgases, um den mikrobiologischen Abbauprozeß am Laufen zu halten, und müssen mit Heizöl oder Erdgas zuheizen.

Eine Anordnung der eingangs genannten Art ist aus der DE 31 38 452 A1 bekannt geworden. Der dort verwendete Faulbehälter kann organische Abfälle, auch Klärschlamm aufnehmen. Der Faulbehälter wird von der Abwärme eines vom anfallenden Faulgas betriebenen Verbrennungsmotors und/oder eines Generators beheizt, so daß die erforderliche Faultemperatur auch bei der chargenweisen Zugabe neuen Faulguts erreicht wird. Unterhalb der idealen Faultemperatur entsteht hier recht wenig Faulgas. Auch hängt ein ausreichender Energiegewinn von der Zusammensetzung der organischen Abfälle ab, mit denen der Faulbehälter beschickt wird.

Eine mehrstufige Anlage samt Prozess zur Behandlung von Klärschlamm geht aus der DE 101 07 712 A1 hervor. Dort sind zwei hintereinander geschaltete Faulbehälter vorgesehen, von denen der erste auf etwa 56 °C aufgeheizt wird. Wie die Aufheizung erfolgt, geht aus dieser Druckschrift nicht hervor.

Ebenfalls mehrstufig ist ein aus der US 2003/0 173 291 A1 bekanntes Verfahren mit Anlage zur Behandlung von Biomasse, bei welcher die zweite Stufe einen Reaktor umfasst, der abwechselnd mit Unter- und Überdruck beaufschlagt wird, wodurch sich der Aufschluss von Zellulose verbessern und so die Energieausbeute erhöhen soll. Hier ist die Biogaserzeugung stark zeitabhängig.

Es ist daher Aufgabe der vorliegenden Erfindung ein wirtschaftlicheres und zugleich umweltfreundliches Verfahren zur Behandlung von Klärschlamm sowie eine Anordnung zur Durchführung des Verfahrens anzugeben.

Die beim Wärme-Kraft-Prozeß anfallende Abwärme kann vorteilhaft zusätzlich zur thermischen Desintegration des Klärschlamms verwendet wird. Dadurch wird die mikroorganische Umsetzung verbessert und die Ausbeute an Klärgas erhöht.

Zusätzlich kann die beim Wärme-Kraft-Prozeß anfallende Abwärme auch zur Trocknung des stabilisierten Klärschlamms verwendet werden. Dies vereinfacht Transport und Deponierung.

Die erfindungsgemäße Anordnung umfaßt eine Kläranlage und eine Biogasanlage. Die Kläranlage besitzt einem Faulturm zur anaeroben Stabilisierung anfallenden Klärschlamms. Die Biogasanlage besitzt einen Fermenter zur Produktion von Biogas aus nachwachsenden Rohstoffen sowie eine Wärmekraftmaschine und einen Generator zur Verstromung des erzeugten Biogases. Erfindungemäß ist eine Wärmeleitung von der Wärmekraftmaschine zum Faulturm vorgesehen, durch die anfallende Abwärme dem Faulturm zur Unterstützung der anaeroben Stabilisierung zuführbar ist.

Vorzugsweise ist außerdem eine Gasleitung vom Faulturm der Kläranlage zur Wärmekraftmaschine der Biogasanlage vorgesehen ist, durch die im Faulturm anfallendes Klärgas der Wärmekraftmaschine zur Verstromung zuführbar ist.

Zweckmäßig ist in der Gasleitung ein Gaszähler vorgesehen. Dies gestattet einen wirtschaftlich unabhängigen Betrieb der zwei Anlagen und erlaubt eine getrennte Abrechnung der erzeugten und verbrauchten Energiemengen. Außerdem ermöglicht eine Feststellung der erzeugten Energiemengen auch eine Optimierung der ablaufenden Prozesse.

Vorteilhaft können in der Kläranlage auch noch eine Vorrichtung zur thermischen Desintegration des Klärschlamms und/oder eine Vorrichtung zur Trocknung des stabilisierten Klärschlamms vorgesehen sein, die über eine Wärmeleitung mit Abwärme der Wärmekraftmaschine versorgt werden.

Im folgenden wird ein Ausführungsbeispiel der Erfindung anhand der Zeichnungen näher erläutert. Es zeigen:
- Figur 1: ein Blockdiagramm der erfindungsgemäßen Anordnung und
- Figur 2: ein detaillierteres Blockdiagramm der Biogasanlage.

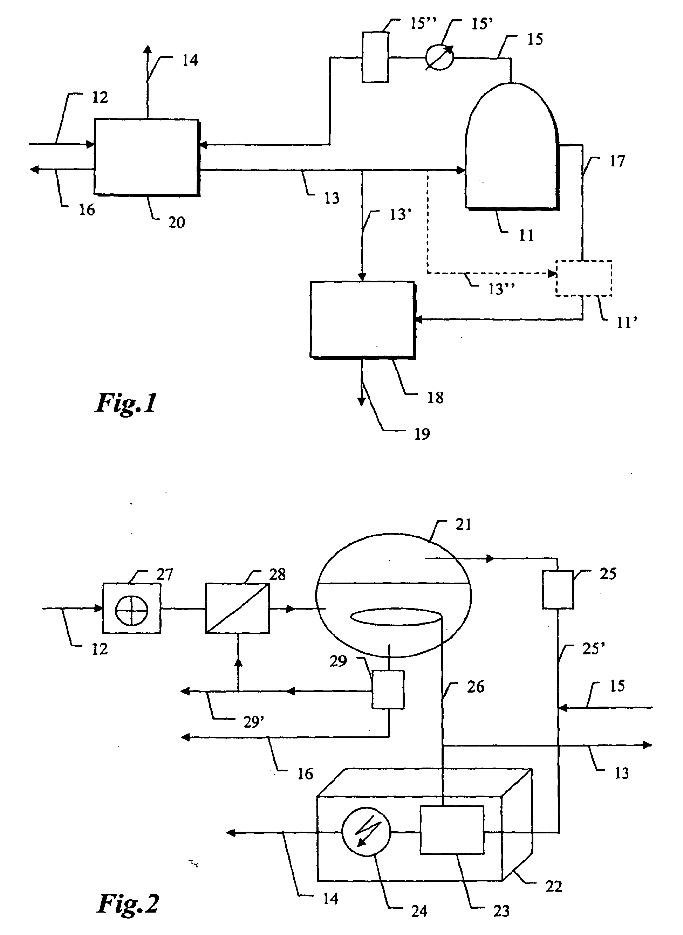

In Figur 1 ist in einem Blockdiagramm dargestellt, wie eine Biogasanlage 20 und der Faulturm 11 einer Kläranlage zusammenwirken. In der Biogasanlage werden nachwachsende Rohstoffe 12, sogenannte Nawaros, in einem Gärprozeß unter Bildung von Biogas vergoren, daß dann in einem Blockheizkraftwerk verstromt wird. Die Biogasanlage 20 produziert somit Strom 14 und Wärme. Ein Teil der Wärme wird in der Biogasanlage 20 selbst benötigt. Der größte Teil der Wärme steht jedoch als Abwärme zur Verfügung, die über eine Wärmeleitung 13, beispielsweise eine Warmwasserleitung, zur Erwärmung von Klärschlamm in den Faulturm 11 geleitet wird. Verbleibende Gärreste 16 aus der Biogasanlage 20 können als Dünger genutzt werden.

In dem Faulturm 11 wird Klärschlamm in einem anaeroben Prozeß durch Mikroorganismen verfault, wobei Klärgas entsteht. Das Klärgas wird über eine Gasleitung 15 mit Gaszähler 15' und Gasaufbereitungsstufe 15'' dem Blockheizkraftwerk der Biogasanlage 20 zugeführt. Selbstverständlich kann das Klärgas alternativ auch in einem eigenen Blockheizkraftwerk in der Kläranlage genutzt werden.

Ausgefaulter Klärschlamm 17 wird zu einer Trocknungsanlage 18 transportiert, die ebenfalls über eine Wärmeleitung 13' mit Abwärme aus der Biogasanlage 20 betrieben wird. Der fertig getrocknete Klärschlamm 19 wird dann einer Deponie zugeführt. Eine optionale, zusätzliche Anlage zur thermischen Desintegration 11' des ausgefaulten Klärschlamms 17 und eine Wärmeleitung 13'' sind gestrichelt gezeigt.

Die Funktionsweise der Biogasanlage 20 ist in Figur 2 näher gezeigt. Herz der Biogasanlage ist ein Fermenter 21, das ist ein Reaktor, in dem aus Biomaterial in feuchter Atmosphäre unter Luftabschluß durch mehrstufige mikrobielle Abbaureaktionen sogenanntes Biogas entsteht. Biogas besteht zu etwa 50 bis 70% aus Methan (CH₄) und zu etwa 30 bis 50% aus Kohlendioxid (CO₂). Andere Gasbestandteile wie Sauerstoff, Stickstoff, Ammoniak, Wasserstoff, Kohlenmonoxid und Schwefelwasserstoff sind nur in geringen Konzentrationen im Biogas enthalten und machen in Summe meist weniger als 1 Vol.-% der anfallenden Gasmenge aus. Als Biomaterial für die Vergärung kommen hier Nawaros wie z.B. Mais, Gras, Raps, oder Grünabfälle zum Einsatz.

Angeliefertes Biomaterial 12 wird von Fremdstoffen gereinigt und in einem Häcksler 27 zerkleinert. Dann gelangt es in einen Annahmebunker (nicht gezeigt) der Biogasanlage 20. Förderbänder transportieren das Biogut in einen Dosierer 28, in welchem es mit zückgeführtem Prozeßwasser sowie bereits vergorenem Material vermischt wird. Dieses "Impfmaterial" beschleunigt den Gärprozeß. Eine Pumpe befördert das befeuchtete Biomaterial in den Fermenter 21.

Über ca. 2 Wochen durchströmt das Biogut den Fermenter 21. Das beim Gärprozeß entstehende Biogas wird gesammelt, in einer Gasaufbereitungsstufe 25 gereinigt, entfeuchtet und ohne Zwischenspeicherung über eine Gasleitung 25' in das Blockheizkraftwerk 22 geführt, welches eine Wärmekraftmaschine wie hier einen Gasmotor 23 und einen mit diesem gekoppelten Generator 24 enthält. Neben dem Biogas aus dem Fermenter 21 wird dem Gasmotor 23 auch das ebenfalls methanhaltige Klärgas aus dem Faulturm 11 der Kläranlage über eine Gasleitung 15 mit Gasaufbereitungsstufe 15'' und Gaszähler 15' zur Verbrennung zugeführt.

Etwa gut ein Drittel der Energie aus der Verbrennung des Biogases wird in dem Blockheizkraftwerk 22 in elektrische Energie umgewandelt. Knapp zwei Drittel der Energie stehen dagegen nur in Form von Wärmeenergie zur Verfügung. Ein Teil der Wärmeenergie wird benötigt, um über die Warmwasserleitung 26 den Fermenter 21 auf Prozeßtemperatur von etwa 55°C zu beheizen. Zusätzlich kann das frische Biomaterial vor dem Einfüllen noch über einen Wärmetauscher (nicht gezeigt) vorgewärmt werden.

Der größte Teil der Wärme steht als Abwärme zur Verfügung. Während die Abwärme bei konventionellen Biogasanlagen meist mangels Abnehmer ungenutzt an die Umgebungsluft abgegeben wird, nutzt die Erfindung die Abwärme zur Unterstützung der anaeroben Stabilisierung von Klärschlamm in der Kläranlage. Der über den Eigenverbrauch der Biogasanlage 20 hinaus erzeugte Strom 14 wird in das öffentliche Elektrizitätsnetz eingespeist.

Aus dem Fermenter 21 wird der Gärstoffrest mittels einer Austrags- und Preßschnecke 29 entfernt und entwässert. Ein Teil des Preßwassers wird für die Befeuchtung des neuen Biomaterials im Dosierer 28 benötigt, der Rest des Preßwassers 29' kann entweder in der Landwirtschaft als Dünger eingesetzt werden oder, da keinerlei umweltschädliche Stoffe in der Biogasanlage verwendet werden, direkt der Kläranlage zugeführt werden.

Der trockene Gärrest 16 wird in einen Nachrottebunker (nicht gezeigt) befördert, wo er unter Einwirkung von Luftsauerstoff kompostiert, bevor er als begehrter Dünger in der Landwirtschaft eingesetzt wird.

Die Dimensionierung der Biogasanlage 20 (z.B. 250 kW, 500kW oder 1000kW) erfolgt nach dem Bedarf der Kläranlage. Hauptsächlich dient die Abwärme der Biogasanlage 20 zum thermophilen Betrieb der im Faulturm 11 ablaufenden anaeroben Stabilisierung insbesondere während der Wintermonate. Allerdings kann die Abwärme nicht nur zur Beheizung des Faulturmes 11 eingesetzt werden, sondern überdies auch auf andere Weise zur Optimierung der Arbeitsweise der Kläranlage nutzbar gemacht werden. Zum einen kann die Abwärme wie im Ausführungsbeispiel auch vorteilhaft zur Trocknung des ausgefaulten Klärschlamms verwendet werden. Auf der anderen Seite kann mit der Abwärme, wie in Figur 1 gestrichelt angedeutet, in einem Nachbehandlungsschritt eine thermische Desintegration des Klärschlamms bei ca. 70-80°C durchgeführt werden.

Dabei durchläuft der dann bereits vorgewärmte, pumpfähige und ausreichend homogenisierte Klärschlamm nach der Vergärung im Faulturm 11 eine thermische Desintegrationsanlage 11' .

Bei der mesophilen Faulung im Faulturm 11 werden bereits 70 bis 80 % der üblichen Klärgasausbeute freigesetzt. Durch eine weitere Faulung ließe sich ohne die thermische Behandlung in der Anlage 11', im Verhältnis zum dafür benötigten Behältervolumen, nur noch relativ wenig Gasertrag erzielen.

Durch die thermische Desintegration bei 70-80°C erfolgt ein Zellaufschluß der mesophilen Bakterienstämme aus der ersten Faulung und aufgrund der Zerstörung der noch vorhandenen Krankheitskeime und Unkrautsamen wird wieder organisches Material freigesetzt. Außerdem wird die Zellstruktur schwer abbaubarer und ansonsten, in vertretbaren Zeiträumen nicht für den Abbauprozeß verfügbarer Stoffe zerstört. Diese werden aufgeschlossen, hydrolysiert und so deren weitere Vergärung auch bei kurzen Verweilzeiten ermöglicht. Die Aufheizung erfolgt direkt über eine Heißwasserleitung 13'' mit der Abwärme des Blockheizkraftwerkes 22 (ca. 90°C heißes Wasser). Anschließend wird der desintegrierte Klärschlamm in einen Nachfaulreaktor der Anlage 11' geleitet, wo er in einem zweiten Faulprozeß weiter zersetzt wird.

## Patentansprüche

1. Verfahren zur umweltverträglichen Behandlung von
Klärschlamm, bei dem der Klärschlamm in einem Faulturm (11) unter Verwendung anaerober Mikroorganismen unter zumindest überwiegendem Sauerstoffabschluss stabilisiert wird, wobei
dem Faulturm (11) über eine Wärmeleitung (13) Abwärme zur Unterstützung der anaeroben Stabilisierung zugeführt wird,
**dadurch gekennzeichnet, dass**
in einem zusätzlichen separaten Fermenter (21) unter Verwendung anaerober Mikroorganismen aus nachwachsenden Rohstoffen Biogas erzeugt und in einem Wärme-Kraft-Prozess verstromt wird, und
beim Wärme-Kraft-Prozess anfallende Abwärme dem Faulturm (11) über die Wärmeleitung (13) zur Unterstützung der anaeroben Stabilisierung zugeführt wird.

2. Verfahren nach Anspruch 1, **dadurch gekennzeichnet,**
**daß** im Faulturm (11) anfallendes Klärgas dem Wärme-Kraft-Prozeß zur Verstromung zugeführt wird.

3. Verfahren nach einem der vorhergehenden Ansprüche,
**dadurch gekennzeichnet, daß** die beim Wärme-KraftProzeß anfallende Abwärme zusätzlich zur thermischen Desintegration des Klärschlamms verwendet wird.

4. Verfahren nach einem der vorhergehenden Ansprüche,
**dadurch gekennzeichnet, daß** die beim Wärme-KraftProzeß anfallende Abwärme zusätzlich zur Trocknung des stabilisierten Klärschlamms verwendet wird.

5. Anordnung umfassend eine Kläranlage mit einem Faulturm (11) zur anaeroben Stabilisierung anfallenden Klärschlamms, eine Wärmekraftmaschine (23) und einen Generator (24), wobei eine Wärmeleitung (13) von der Wärmekraftmaschine (23) zum Faulturm (11) vorgesehen ist, durch die anfallende Abwärme dem Faulturm (11) zur Unterstützung der anaeroben Stabilisierung zuführbar ist,
**dadurch gekennzeichnet, dass**
die Anordnung außerdem eine separate Biogasanlage (20) mit einem Fermenter (21) zur Produktion von Biogas aus nachwachsenden Rohstoffen (12) aufweist, wobei
die Wärmekraftmaschine (23) und der Generator (24) zur Verstromung des erzeugten Biogases vorgesehen sind.

6. Anordnung nach Anspruch 5, **dadurch gekennzeichnet,**
**daß** eine Gasleitung (15) vom Faulturm der Kläranlage zur Wärmekraftmaschine (23) der Biogasanlage (20) vorgesehen ist, durch die im Faulturm (11) anfallendes Klärgas der Wärmekraftmaschine (23) zur Verstromung zuführbar ist.

7. Anordnung nach Anspruch 6, **dadurch gekennzeichnet,**
**daß** in der Gasleitung (15) ein Gaszähler (15') vorgesehen ist.

8. Anordnung nach einem der Ansprüche 5 bis 7, **dadurch**
**gekennzeichnet, daß** in der Kläranlage eine Vorrichtung zur thermischen Desintegration (11') des Klärschlamms vorgesehen ist, die über die Wärmeleitung (13'') mit Abwärme der Wärmekraftmaschine (23) versorgt ist.

9. Anordnung nach einem der Ansprüche 5 bis 8, **dadurch**
**gekennzeichnet, daß** in der Kläranlage eine Vorrichtung (18) zur Trocknung des stabilisierten Klärschlamms vorgesehen ist, die über die Wärmeleitung (13') mit Abwärme der Wärmekraftmaschine (23) versorgt ist.

## Claims

1. Process for the environmentally compatible treatment of sewage sludge in which the sewage sludge is stabilised in a digester (11) using anaerobic microorganisms with at least predominant exclusion of oxygen, wherein
waste heat is fed to the digester (11) via a heat pipe (13) to support the anaerobic stabilisation,
**characterised in that**
biogas is produced in an additional separate fermenter (21) using anaerobic microorganisms from renewable raw materials and converted to electricity in a thermal power process, and
waste heat produced in the thermal power process is fed to the digester (11) via the heat pipe (13) to support the anaerobic stabilisation.

2. Process according to claim 1, **characterised in that** digester gas produced in the digester (11) is fed to the thermal power process for conversion to electricity.

3. Process according to one of the preceding claims, **characterised in that** the waste heat produced in the thermal power process is used in addition for the thermal disintegration of the sewage sludge.

4. Process according to one of the preceding claims, **characterised in that** the waste heat produced in the thermal power process is used in addition for drying the stabilised sewage sludge.

5. Arrangement comprising a waste water treatment plant with a digester (11) for the anaerobic stabilisation of sewage sludge produced, a combustion engine (23) and a generator (24), wherein a heat pipe (13) from the combustion engine (23) to the digester (11) is provided, through which the waste heat produced can be fed to the digester (11) to support the anaerobic stabilisation,
**characterised in that**
the arrangement furthermore has a separate biogas plant (20) with a fermenter (21) for the production of biogas from renewable raw materials (12), wherein
the combustion engine (23) and the generator (24) are provided to convert the biogas produced to electricity.

6. Arrangement according to claim 5, **characterised in that** a gas pipe (15) from the digester of the waste water treatment plant to the combustion engine (23) of the biogas plant (20) is provided, through which digester gas produced in the digester (11) can be fed to the combustion engine (23) for conversion to electricity.

7. Arrangement according to claim 6, **characterised in that** a gas meter (15') is provided in the gas pipe (15).

8. Arrangement according to one of claims 5 to 7, **characterised in that** a device for the thermal disintegration (11') of the sewage sludge which is supplied via the heat pipe (13") with waste heat from the combustion engine (23), is provided in the waste water treatment plant.

9. Arrangement according to one of claims 5 to 8, **characterised in that** a device (18) for drying the stabilised sewage sludge which is supplied via the heat pipe (13') with waste heat from the combustion engine (23), is provided in the waste water treatment plant.

## Revendications

1. Procédé pour traiter, en respectant l'environnement, des boues de curage, selon lequel les boues de curage sont stabilisées dans une tour de digestion (11) en utilisant des microorganismes anaérobies pour l'essentiel en l'absence d'oxygène,
de la chaleur perdue étant apportée via une conduite de chaleur (13) à la tour de digestion (11) afin d'aider à la stabilisation anaérobie,
**caractérisé en ce que**
du biogaz est produit dans un fermenteur distinct supplémentaire (21), en utilisant des microorganismes anaérobies, à partir de matières premières renouvelables, et il est transformé en courant électrique dans un processus de production combinée de chaleur et d'électricité,
et la chaleur perdue produite lors du processus de production combinée de chaleur et d'électricité est apportée à la tour de digestion (11) via la conduite de chaleur (13) afin d'aider à la stabilisation anaérobie.

2. Procédé selon la revendication 1, **caractérisé en ce que** le gaz de curage produit dans la tour de digestion (11) est apporté au processus de production combinée de chaleur et d'électricité pour la transformation en courant électrique.

3. Procédé selon l'une des revendications précédentes, **caractérisé en ce que** la chaleur perdue produite lors du processus de production combinée de chaleur et d'électricité est en outre utilisée pour la désintégration thermique des boues de curage.

4. Procédé selon l'une des revendications précédentes, **caractérisé en ce que** la chaleur perdue produite lors du processus de production combinée de chaleur et d'électricité est en outre utilisée pour le séchage des boues de curage stabilisées.

5. Système comprenant une station d'épuration avec une tour de digestion (11) pour la stabilisation anaérobie des boues de curage produites, une machine (23) de production d'énergie électrique par voie thermique et un générateur (24), sachant qu'il est prévu une conduite de chaleur (13) allant de la machine (23) de production d'énergie électrique par voie thermique à la tour de digestion (11), conduite par laquelle la chaleur perdue produite peut être apportée à la tour de digestion (11) afin d'aider à la stabilisation anaérobie,
**caractérisé en ce que**
le système comprend en outre une installation séparée (20) de production de biogaz avec un fermenteur (21) pour produire du biogaz à partir de matières premières renouvelables (12),
sachant que la machine (23) de production d'énergie électrique par voie thermique et le générateur (24) sont prévus pour transformer en courant électrique le biogaz produit.

6. Système selon la revendication 5, **caractérisé en ce qu'**il est prévu une conduite de gaz (15) allant de la tour de digestion de la station d'épuration à la machine (23) de production d'énergie électrique par voie thermique de l'installation (20) de production de biogaz, conduite par laquelle le gaz de curage produit dans la tour de digestion (11) peut être apporté à la machine (23) de production d'énergie électrique par voie thermique pour la transformation en courant électrique.

7. Système selon la revendication 6, **caractérisé en ce qu'**un compteur à gaz (15') est prévu dans la conduite de gaz (15).

8. Système selon l'une des revendications 5 à 7, **caractérisé en ce qu'**un dispositif pour la désintégration thermique (11') des boues de curage est prévu dans la station d'épuration, dispositif qui est alimenté via la conduite de chaleur (13") en chaleur perdue de la machine (23) de production d'énergie électrique par voie thermique.

9. Système selon l'une des revendications 5 à 8, **caractérisé en ce qu'**un dispositif (18) pour le séchage des boues de curage stabilisées est prévu dans la station d'épuration, dispositif qui est alimenté via la conduite de chaleur (13') en chaleur perdue de la machine (23) de production d'énergie électrique par voie thermique.
